(19) Europäisches Patentamt
European Patent Office
Office européen des brevets

(11) Veröffentlichungsnummer: **0 193 131**
**B1**

(12) # EUROPÄISCHE PATENTSCHRIFT

(45) Veröffentlichungstag der Patentschrift: **06.12.89**

(51) Int. Cl.⁴: **C 07 D 261/18,** A 01 N 43/80

(21) Anmeldenummer: **86102281.2**

(22) Anmeldetag: **21.02.86**

(54) **5-Isoxazolcarbonsäureamide, ihre Herstellung und Verwendung als Fungizide.**

(30) Priorität: **27.02.85 DE 3506814**

(43) Veröffentlichungstag der Anmeldung: **03.09.86 Patentblatt 86/36**

(45) Bekanntmachung des Hinweises auf die Patenterteilung: **06.12.89 Patentblatt 89/49**

(84) Benannte Vertragsstaaten: **AT BE CH DE FR GB IT LI NL SE**

(56) Entgegenhaltungen:
**EP-A-0 012 428**
**EP-A-0 012 435**
**EP-A-0 026 873**
**EP-A-0 037 927**
**EP-A-0 078 999**
**EP-A-0 080 080**
**DE-B-1 670 945**

(73) Patentinhaber: **BASF Aktiengesellschaft, Carl-Bosch- Strasse 38, D-6700 Ludwigshafen (DE)**

(72) Erfinder: **Buschmann, Ernst, Dr., Georg- Ludwig-Krebs- Strasse 10, D-6700 Ludwigshafen (DE)**
Erfinder: **Rentzea, Costin, Dr., Richard- Kuhn-Strasse 1-3, D-6900 Heidelberg (DE)**
Erfinder: **Roeser, Karl, Dr., Muldweg 11, D-6945 Hirschberg (DE)**
Erfinder: **Sauter, Hubert, Dr., Neckarpromenade 20, D-6800 Mannheim 1 (DE)**
Erfinder: **Spiegler, Wolfgang, Dr., Westpreussenstrasse 5, D-6520 Worms 27 (DE)**
Erfinder: **Ammermann, Eberhard, Dr., Sachsenstrasse 3, D-6700 Ludwigshafen (DE)**
Erfinder: **Pommer, Ernst- Heinrich, Dr., Berliner Platz 7, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 193 131 B1

LIBER, STOCKHOLM 1989

**Beschreibung**

Die vorliegende Erfindung betrifft neue wertvolle 5-Isoxazolcarbonsäureamide mit guter fungizider Wirkung und ihre Anwendung als Fungizide.

Es ist bekannt, Isoxazolcarbonsäureamide, z. B. das Isoxazol-5-carbonsäure-N-(1-methoxycarbonyl-ethyl)-(2,6-dimethylanilid) (DE-2 940 189) als Fungizide zu verwenden. Die Wirkung dieser Verbindungen ist jedoch vor allem bei niedrigen Aufwandmengen nicht befriedigend. Eine Aufgabe der vorliegenden Erfindung ist es daher, neue Verbindungen und Fungizide mit hoher fungitoxischer Wirkung bei gleichzeitig guter Pflanzenverträglichkeit bereitzustellen.

Es wurde nun gefunden, daß die 5-Isoxazolcarbonsäureamide der Formel I

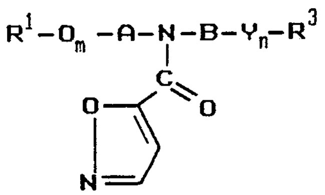

(I)

in der

$R^1$ einen gegebenenfalls ein- bis dreifach durch Halogen, $C_1$-$C_5$-Alkyl, $C_1$-$C_2$-Alkoxy, $C_1$-$C_2$-Alkylthio $C_1$-$C_2$-Halogenalkyl, Nitro, Phenyl, Halogenphenyl, Phenoxy, Halogenphenoxy substituierten Phenylrest oder einen gegebenenfalls ein- bis dreifach durch $C_1$-$C_3$-Alkyl substituierten $C_1$-$C_8$-Alkylrest, einen gegebenenfalls ein- bis dreifach durch $C_1$-$C_3$-Alkyl substituierten $C_3$-$C_8$-Cycloalkylrest oder einen gegebenenfalls ein- bis dreifach durch Halogen oder $C_1$-$C_4$-Alkyl substituierten Benzylrest bedeutet;

Y ein Sauerstoffatom oder die Gruppe $NR^2$ bedeutet,
$R^2$ einen verzweigten oder unverzweigten Alkylrest mit 1 bis 4 C-Atomen bedeutet;
m und
n unabhängig voneinander für die Zahlen 0 und 1 stehen:
A für einen unverzweigten oder verzweigten Alkylenrest mit 2 bis 10 Kohlenstoffatomen steht;
B für einen verzweigten oder unverzweigten Alkylenrest mit einem bis 10 Kohlenstoffatomen oder einen Cycloalkylenrest mit 5 bis 8 Kohlenstoffatomen steht und
$R^3$ einen Phenylrest, einen Halogenphenylrest, einen verzweigten oder unverzweigten Alkylrest mit 1 bis 10 Kohlenstoffatomen oder ein Wasserstoffatom bedeutet,

bei ausgezeichneter Pflanzenverträglichkeit eine hohe fungitoxische Wirkung besitzen.

Die neuen Verbindungen Kannen gegebenenfalls ein oder mehrere Chiralitätszentren enthalten und dann als Diastereomeren- und/oder Enantiomerengemische vorliegen. Zur Gewinnung der reinen Stereoisomeren können entweder die Isomerengemische durch geeignete Methoden, z. B. Chromatographie an chiralen stationären Phasen oder durch fraktionierte Kristallisation, getrennt werden, oder sie können durch geeignete Reaktionsführung direkt, z. B. durch die Verwendung homochiraler Ausgangsmaterialien, in isomerenreiner Form erhalten werden. Die neuen Verbindungen können auch in Form von cis- oder trans-Isomeren vorliegen. Alle diese Isomeren und ihre Mischungen werden von der vorliegenden Erfindung umfaßt.

In Formel I bedeuten beispielsweise

$R^1$gegebenenfalls ein- bis dreifach substituierte Phenylreste wobei die Substituenten aus der folgenden Gruppe ausgewählt sein können:

Halogen, Alkyl, Alkoxy, Alkylthio, Halogenalkyl, Nitro, Cyano, ggf. substituiertes Phenyl (Halogenphenyl), ggf. substituiertes Phenoxy (Halogenphenoxy) oder Wasserstoff.

Beispiele für Halogen sind Fluor, Chlor und Brom, wobei Fluor und Chlor bevorzugt sind. Besonders bevorzugt sind die Substitutionsmuster 2-F; 2-F, 4-Cl; 4-Cl, 2,4-$Cl_2$, 2,4,6-$Cl_3$.

Beispiele für Alkyle sind verzweigte oder unverzweigte Alkylreste mit 1 bis 5 Kohlenstoffatomen, z. B. Methyl, Ethyl, n-Propyl, i-Propyl, n-Butyl, t-Butyl, n-Pentyl, i-Pentyl oder neo-Pentyl, wobei Methyl und t-Butyl bevorzugt sind.

Beispiele für Alkoxy sind Methoxy und Ethoxy.

Beispiele für Alkylthio sind Methylthio und Ethylthio.

Beispiele für Halogenalkylreste sind beispielsweise Halogenalkylreste mit 1 oder 2 Kohlenstoffatomen und einem, 2 oder 3 gleichen oder verschiedenen Halogenatomen, wobei als Halogen vorzugsweise Fluor und Chlor geeignet sind und als Beispiel für Halogenalkyl Trifluormethyl genannt wird.

Weiterhin kann $R^1$ für einen gegebenenfalls ein- bis dreifach substituierten Alkyl- oder Cycloalkylrest stehen, wobei als Alkylreste unverzweigtes $C_1$-$C_8$-Alkyl und als Cycloalkylreste $C_3$- bis $C_8$-Cycloalkyl z. B. Cyclopropyl, Cyclobutyl, Cyclopentyl, Cyclohexyl, Cycloheptyl und Cyclooctyl in Betracht kommen und Cyclohexylreste bevorzugt sind. Als Substituenten dieser Alkyl- oder Cycloalkylreste kommen verzweigte und unverzweigte Alkylreste

mit bis zu 3 Kohlenstoffatomen in Betracht, z. B. Methyl, Ethyl, n-Propyl und Isopropyl.

Außerdem kann $R^1$ für einen gegebenenfalls ein- bis dreifach substituierten Benzylrest stehen, z. B. Halogenbenzyl oder $C_1$- bis $C_4$-Alkylbenzyl beispielsweise 4-Cl-Benzyl, 2,4-Dichlorbenzyl, 2,4,6-Trichlorbenzyl, 4-tert.-Butylbenzyl oder 4-Fluorbenzyl.

Y steht in den Verbindungen der Formel I für Sauerstoffatome, wobei Verbindungen mit einem Sauerstoffatom bevorzugt werden; außerdem steht Y in der Formel I für die Gruppe $NR^2$, wobei $R^2$ einen verzweigten oder unverzweigten Alkylrest mit 1 bis 4 C-Atomen bedeutet. $R^2$ steht dabei beispielsweise für Methyl, Ethyl, n-Propyl oder i-Propyl, n-Butyl, wobei Methyl und Ethyl bevorzugt sind.

A steht in Formel I für verzweigte oder unverzweigte Alkylenreste mit 2 bis 10 Kohlenstoffatomen wie Ethan-1,2-diyl, n-Propyl-1,3-diyl, n-Butan-1,4-diyl, 2-Methyl-propan-1,3-diyl, n-Pentan-1,5-diyl, n-Heyan-1,6-diyl, n-Heptan-1,7-diyl, n-Octan-1,8-diyl, n-Nonan-1,9-diyl oder n-Decan-1,10-diyl, wobei solche Verbindungen bevorzugt sind, die zwei bis sechs Kohlenstoffatome im Rest A enthalten und als Beispiele Ethan-1,2-diyl, n-Propan-1,3-diyl, n-Butan-1,4-diyl, 2-Methyl-propan-1,3-diyl, n-Pentan1,5-diyl oder n-Hexan-1,6-diyl genannt werden.

B steht in Formel I für verzweigte oder unverzweigte Alkylenreste mit einem bis 10 Kohlenstoffatomen, beispielsweise Methylen, Ethan-1,2-diyl, 2-Methyl-ethan-1,2-diyl, n-Propan-1,3-diyl, n-Propan-2,2-diyl, n-Butan-1,4-diyl, 2-Methyl-propan-1,3-diyl, 2-Methylpropan-2,3-diyl, n-Pentan-1,5-diyl, n-Hexan-1,6-diyl, n-Heptan-1,7-diyl oder n-Octan-1,8-diyl, n-Nonan-1,9-diyl oder n-Decan-1,10-diyl, oder Cycloalkylenreste mit 5 bis 8 Kohlenstoffatomen z. B. Cyclohexan-1,4-diyl, wobei solche Alkylenreste bevorzugt sind, die 2 bis 6 Kohlenstoffatome im Rest B enthalten und als Beispiele Ethan-1,2-diyl, n-Propan-1,3-diyl, n-Butan-1,4-diyl, 2-Methylpropan-1,3-diyl, n-Pentan-1,5-diyl, oder n-Hexan-1,6-diyl genannt werden.

$R^3$ steht in Formel I für einen Phenylrest, einen Halogenphenylrest einen verzweigten oder unverzweigten Alkylrest mit 1 bis 10 Kohlenstoffatomen sowie für Wasserstoff. Beispiele für substituierte Phenylreste sind Halogenphenyl, 4-Cl-Phenyl, 2,4-Dichlorphenyl, 2-Fluorphenyl, 4-Fluorphenyl, 2,4,6-Trichlorphenyl, Halogenalkylphenyl, 2-Trifluormethylphenyl, 4-Trifluormethylphenyl. Beispiele für Alkylreste sind Methyl, Ethyl, n-Propyl, i-Propyl, n-Butyl, t-Butyl, n-Pentyl, i-Pentyl, neo-Pentyl, n-Hexyl, n-Heptyl, n-Octyl, n-Nonyl und n-Decyl, wobei $C_1$-$C_4$-Alkyl, Methyl, n-Butyl, t-Butyl und sec.-Butyl bevorzugt sind.

Weiterhin wurde gefunden, daß man die neuen 5-Isoxazolcarbonsäureamide erhält, wenn man Amine der allgemeinen Formel II

$R^1$-$O_m$-A-N-B-$Y_n$-$R^3$ (II),
|
H

in der $R^1$, A, B, Y, $R^3$, m und n die oben genannte Bedeutung haben, mit einem Isoxazolcarbonsäurederivat der Formel III umsetzt,

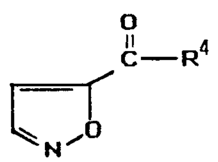
(II)

in der $R^4$ die Halogenatome Chlor oder Brom oder einen Rest der Formel $-ZR^5$ bedeutet, wobei $R^5$ für einen Alkylrest mit einem bis drei Kohlenstoffatomen wie z. B. Methyl, Ethyl, oder n-Propyl steht und Z für ein Sauerstoffatom oder die Gruppe -O-CO- steht. Bevorzugt sind solche Verbindungen der Formel III, in denen $R_4$ für Chlor steht.

Die Herstellung des Wirkstoffs kann ggf. in Gegenwart eines Lösungs- oder Verdünnungsmittels und/oder Katalysatoren und/oder einer Hilfsbase erfolgen. Die Reaktionstemperaturen können zwischen 0°C und 100°C, vorzugsweise zwischen 70°C und 90°C liegen. Als Lösungs- oder Verdünnungsmittel kommen beispielsweise Toluol, Benzol, Cyclohexan, Tetrahydrofuran, Diethylether, Methyldiisopropylether oder Wasser in Betracht, wobei Toluol bevorzugt ist. Als Hilfsbasen bzw. Katalysatoren kommen organische oder anorganische basische Verbindungen in Betracht z. B. Pyridin, Triethylamin, Methylisopropylamin, Kaliumcarbonat, Natriumcarbonat oder Mischungen dieser Verbindungen.

Eine besonders bevorzugte Variante des Herstellungsverfahrens besteht darin, zur Lösung eines Isoxazolsäurederivates der Formel III, in der $R^4$ die oben genannte Bedeutung besitzt, eine Mischung eines sekundären Amins der Formel II mit Pyridin zuzutropfen und den Reaktionsansatz nach Abklingen der Reaktion und ggf. erforderlichem Erhitzen in an sich bekannter Weise aufzuarbeiten.

Die als Ausgangssubstanzen dienenden Amine der Formel II sind entweder bekannt oder lassen sich nach bekannten Verfahren, z. B. Houben-Weyl, Methoden der organischen Chemie, Bd: XI/1, S. 37 ff, Stuttgart 1957 darstellen.

Die Herstellung der neuen Verbindungen wird durch folgende Beispiele erläutert:

**Beispiel 1**

Isoxazol-5-carbonsäure-N,N-dibutylamid

Man löst 7,4 g (0,06 Mol) Isoxazol-5-carbonsäurechlorid in 75 ml trockenem Toluol. Dazu tropft man eine zuvor bereitete Mischung aus 7,2 g (0,06 Mol) Dibutylamin und 9,5 g (0,12 Mol) Pyridin, wobei die Temperatur unterhalb von 50°C gehalten wird. Nach Abklingen der Reaktion rührt man eine Stunde bei 80°C, extrahiert die Toluol-Lösung sechs Mal mit Wasser und trocknet die organische Phase über Magnesiumsulfat. Nach dem Verdampfen des Lösungsmittels verbleiben 16,1 g (94 % d. Th.) eines gelblichen Öls, das nach den spektroskopischen Daten reines Isoxazol-5-carbonsäure-N,N-dibutylamid ist. (Verbindung Nr. 1).

In entsprechender Weise können durch geeignete Variation der Ausgangsmaterialien und/oder der Reaktionsbedingungen die in der folgenden Tabelle aufgeführten Verbindungen hergestellt werden.

| Nr. | R' | m | A | B | n | Y | $R^3$ | Phys. | Daten |
|---|---|---|---|---|---|---|---|---|---|
| 1 | H | 0 | $-(CH_2)_4-$ | $-(CH_2)_4-$ | 0 | | H | Öl | |
| 2 | 2,4-Dichlorphenyl | 0 | 2-Methylpropan-1,3-diyl | $-(CH_2)_6-$ | 0 | | H | Öl | |
| 3 | Phenyl | 1 | $-(CH_2)_4-$ | $-(CH_2)_6-$ | 0 | | H | Öl | |
| 4 | Phenyl | 1 | $-(CH_2)_4-$ | 3-Methyl-5,5-dimethyl-pentyl-1,5-diyl | 0 | | Methyl | Öl | |
| 5 | Phenyl | 1 | $-(CH_2)_4-$ | $-(CH_2)_2-$ | 1 | 0 | n-Butyl | Öl | |
| 6 | Phenyl | 1 | $-(CH_2)_4-$ | $-(CH_2)_3-$ | 1 | 0 | $-CH-(CH_3)_2$ | Öl | |
| 7 | Methyl | 1 | $-(CH_2)_4-$ | $-(CH_2)_4-$ | 0 | | H | Öl | |
| 8 | 4-tert.-Butylphenyl | 0 | $-CH_2-CH(CH_3)-CH_2$ | $-(CH_2)_2-$ | 0 | | H | Öl | |
| 9 | 2,4-Dichlorphenyl | 0 | $-CH_2-CH(CH_3)-CH_2$ | Cyclohexan-1,4-diyl | 0 | | H | Öl | |
| 10 | 4-tert.-Butylphenyl | 0 | $-(CH_2-CH(CH_3)-CH_2$ | $-CH_2-$ | 0 | | H | Öl | |
| 11 | Phenyl | 1 | $-(CH_2)_2-$ | $-(CH_2)_2-$ | 0 | | Methyl | Öl | |
| 12 | Phenyl | 1 | $-(CH_2)_2-$ | $-(CH_2)_5-$ | 0 | | Methyl | Öl | |
| 13 | Phenyl | 1 | $-(CH_2)_2-$ | $-(CH_2)_5-$ | 0 | | Methyl | Öl | |
| 14 | Phenyl | 1 | $-(CH_2)_2-$ | $-(CH_2)_7-$ | 0 | | Methyl | Öl | |
| 15 | Phenyl | 1 | $-(CH_2)_2-$ | $-CH_2-CH(CH_3)-$ | 0 | | Methyl | Öl | |
| 16 | Phenyl | 1 | $-(CH_2)_2-$ | $-C(CH_3)_2-$ | 0 | | Methyl | Fp. | 72 - 74°C |
| 17 | Phenyl | 1 | $-(CH_2)_2-$ | $-CH_2-$ | 0 | | Phenyl | Öl | |
| 18 | Phenyl | 1 | $-(CH_2)_3-$ | $-(CH_2)_2-$ | 0 | | Methyl | Öl | |
| 19 | Phenyl | 1 | $-(CH_2)_3-$ | $-(CH_2)_3-$ | 0 | | Methyl | Öl | |
| 20 | Phenyl | 1 | $-(CH_2)_3-$ | $-(CH_2)_5-$ | 0 | | Methyl | Öl | |
| 21 | Phenyl | 1 | $-(CH_2)_3-$ | $-(CH_2)_7-$ | 0 | | Methyl | Öl | |
| 22 | Phenyl | 1 | $-(CH_2)_3-$ | $-CH_2-CH(CH_3)-$ | 0 | | Methyl | Öl | |
| 23 | Phenyl | 1 | $-(CH_2)_3-$ | $-C(CH_3)_2-$ | 0 | | Methyl | Öl | |
| 24 | Phenyl | 1 | $-(CH_2)_3-$ | $-CH_2-$ | 0 | | Phenyl | Öl | |
| 25 | Phenyl | 1 | $-(CH_2)_4-$ | $-(CH_2)_2-$ | 0 | | Methyl | Öl | |
| 26 | Phenyl | 1 | $-(CH_2)_4-$ | $-(CH_2)_4-$ | 0 | | Methyl | Öl | |
| 27 | Phenyl | 1 | $-(CH_2)_4-$ | $-(CH_2)_5-$ | 0 | | Methyl | Öl | |
| 28 | Phenyl | 1 | $-(CH_2)_4-$ | $-(CH_2)_7-$ | 0 | | Methyl | Öl | |
| 29 | Phenyl | 1 | $-(CH_2)_4-$ | $-CH_2-CH(CH_3)-$ | 0 | | Methyl | Öl | |
| 30 | Phenyl | 1 | $-(CH_2)_4-$ | $-C(CH_3)_2-$ | 0 | | Methyl | Öl | |
| 31 | Phenyl | 1 | $-(CH_2)_4-$ | $-CH_2-$ | 0 | | Phenyl | Fp. | 69 - 70°C |
| 32 | Phenyl | 1 | $-(CH_2)_5-$ | $-(CH_2)_2-$ | 0 | | Methyl | Öl | |
| 33 | Phenyl | 1 | $-(CH_2)_4-$ | $-(CH_2)_3-$ | 0 | | Methyl | Öl | |
| 34 | Phenyl | 1 | $-(CH_2)_4-$ | $-(CH_2)_5-$ | 0 | | Methyl | Öl | |
| 35 | Phenyl | 1 | $-(CH_2)_5-$ | $-(CH_2)_6-$ | 0 | | Methyl | Öl | |
| 36 | Phenyl | 1 | $-(CH_2)_5-$ | $-CH_2-CH(CH_3)-$ | 0 | | Methyl | Öl | |
| 37 | Phenyl | 1 | $-(CH_2)_5-$ | $-C(CH_3)_2-$ | 0 | | Methyl | Öl | |
| 38 | Phenyl | 1 | $-(CH_2)_5-$ | $-CH_2-$ | 0 | | Phenyl | Öl | |
| 39 | Phenyl | 1 | $-(CH_2)_6-$ | $-(CH_2)_2-$ | 0 | | Methyl | Öl | |
| 40 | Phenyl | 1 | $-(CH_2)_6-$ | $-(CH_2)_3-$ | 0 | | Methyl | Öl | |
| 41 | Phenyl | 1 | $-(CH_2)_6-$ | $-(CH_2)_5-$ | 0 | | Methyl | Öl | |
| 42 | Phenyl | 1 | $-(CH_2)_6-$ | $-(CH_2)_7-$ | 0 | | Methyl | Öl | |
| 43 | Phenyl | 1 | $-(CH_2)_6-$ | $-CH_2-CH(CH_3)-$ | 0 | | Methyl | Öl | |
| 44 | Phenyl | 1 | $-(CH_2)_6-$ | $-C(CH_3)_2-$ | 0 | | Methyl | Öl | |
| 45 | Phenyl | 1 | $-(CH_2)_6-$ | $-CH_2-$ | 0 | | Phenyl | Öl | |

| Nr. | R' | m | A | B | n | Y | $R^3$ | Phys. Daten |
|---|---|---|---|---|---|---|---|---|
| 46 | Cyclohexyl | 0 | $-(CH_2)_3-$ | Cyclohexan-1,4-diyl | 0 | | H | Öl |
| 47 | 2,4-Dichlorphenyl | 0 | 2-Methyl-propan-1,3-diyl | $-(CH_2)_3-$ | 0 | | H | Öl |
| 48 | 4-tert.-Butylphenyl | 0 | 2-Methyl-propan-1,3-diyl | $-CH_2-$ | 0 | | Phenyl | Öl |
| 49 | 4-tert.-Butylphenyl | 0 | 2-Methyl-propan-1,3-diyl | $-(CH_2)_5-$ | 0 | | H | Ö |
| 50 | 4-tert.-Butylphenyl | 0 | 2-Methyl-propan-1,3-diyl | $-(CH_2)_2-$ | 0 | | i-Propyl | Öl |
| 51 | 4-tert.-Butylphenyl | 0 | 2-Methyl-propan-1,3-diyl | $-CH_2-$ | 0 | | H | Öl |
| 52 | Phenyl | 1 | $-(CH_2)_6-$ | $-CH_2-$ | 0 | | 2,4-Dichlor-phenyl | |
| 53 | Phenyl | 1 | $-(CH_2)_6-$ | $-CH_2-$ | 0 | | 2-Chlor-phenyl | |
| 54 | Phenyl | 1 | $-(CH_2)_6-$ | $-CH_2-$ | 0 | | 4-Chlor-phenyl | |
| 55 | Phenyl | 1 | $-(CH_2)_6-$ | $-CH_2-$ | 0 | | 4-tert-Butylphenyl | |
| 56 | Phenyl | 1 | $-(CH_2)_6-$ | $-C(CH_3)_2-CH_2-$ | 0 | | tert-Butyl | |
| 57 | Phenyl | 1 | $-(CH_2)_6-$ | $-(CH_2)_2-$ | 0 | | i-Propyl | |
| 58 | Phenyl | 1 | $-(CH_2)_6-$ | $-(CH_2)_2-$ | 1 | $-N(C_2H_5)-$ | Ethyl | |
| 59 | 2-Chlorphenyl | 1 | $-(CH_2)_6-$ | $-CH_2-$ | 0 | | 2,4-Dichlor-phenyl | |
| 60 | 2-Chlorphenyl | 1 | $-(CH_2)_6-$ | $-CH_2-$ | 0 | | 2,4-Dichlor-phenyl | |
| 61 | 2-Chlorphenyl | 1 | $-(CH_2)_6-$ | $-CH_2-$ | 0 | | 4-Chlorphenyl | |
| 62 | 2-Chlorphenyl | 1 | $-(CH_2)_6-$ | $-CH_2-$ | 0 | | 4-tert.-Butyl-phenyl | |
| 63 | 2-Chlorphenyl | 1 | $-(CH_2)_6-$ | $-C(CH_3)_2-CH_2-$ | 0 | | tert.-Butyl | |
| 64 | 2-Chlorphenyl | 1 | $-(CH_2)_6-$ | $-(CH_2)_2-$ | 0 | | i-Propyl | |
| 65 | 2-Chlorphenyl | 1 | $-(CH_2)_6-$ | $-(CH_2)_2-$ | 1 | $-N(C_2H_5)-$ | Ethyl | |
| 66 | Phenyl | 1 | $-(CH_2)_8-$ | $-(CH_2)_2-$ | 0 | | Methyl | Öl |
| 67 | Phenyl | 1 | $-(CH_2)_8-$ | $-(CH_2)_2-$ | 0 | | Ethyl | Öl |
| 68 | Phenyl | 1 | $-(CH_2)_8-$ | $-(CH_2)_2-$ | 0 | | n-Butyl | Öl |
| 69 | Phenyl | 1 | $-(CH_2)_8-$ | $-CH_2-$ | 0 | | Phenyl | Öl |
| 70 | Phenyl | 1 | $-(CH_2)_8-$ | $-CH_2-$ | 0 | | 2,4-Dichlor-phenyl | Öl |
| 71 | Phenyl | 1 | $-(CH_2)_8-$ | $-(CH_2)_2-$ | 1 | $-N(C_2H_5)-$ | Ethyl | |
| 72 | Phenyl | 1 | $-(CH_2)_4-$ | $-CH_2-$ | 0 | | 2,4-Dichlor-phenyl | Öl |
| 73 | 2-Chlorphenyl | 1 | $-(CH_2)_4-$ | $-CH_2-$ | 0 | | i-Propyl | Öl |
| 74 | 2-Chlorphenyl | 1 | $-(CH_2)_4-$ | $-C(CH_3)_2-$ | 0 | | Methyl | Öl |
| 75 | 2-Chlorphenyl | 1 | $-(CH_2)_4-$ | $-CH_2-$ | 0 | | tert.-Butyl | Öl |
| 76 | 2,4,6-Trichlor-phenyl | 1 | $-(CH_2)_4-$ | $-CH_2-$ | 0 | | i-Propyl | Öl |
| 77 | 2,4,6-Trichlor-phenyl | 1 | $-(CH_2)_4-$ | $-C(CH_3)_2-$ | 0 | | Methyl | Öl |
| 78 | 2,4,6-Trichlor-phenyl | 1 | $-(CH_2)_4-$ | $-CH_2-$ | 0 | | tert.-Butyl | Öl |
| 79 | Phenyl | 1 | $-(CH_2)_4-$ | $-(CH_2)_2-$ | 1 | $-N(C_2H_5)-$ | Ethyl | |
| 80 | 2-Chlorphenyl | 1 | $-(CH_2)_4-$ | $-(CH_2)_2-$ | 1 | $-N(C_2H_5)-$ | Ethyl | |
| 81 | 2,4,6-Trichlorphenyl phenyl | 1 | $-(CH_2)_4-$ | $-(CH_2)_2-$ | 1 | $-N(C_2H_5)-$ | Ethyl | |

| Verbindung Nr. | IR (Film) [cm$^{-1}$] | | | | |
|---|---|---|---|---|---|
| 1 | 1643, | 1422, | 2960, | 2934, | 2874 |
| 2 | 1643, | 1474, | 2929, | 1421, | 2957 |
| 3 | 1643, | 1245, | 2930, | 1422, | 1498 |
| 4 | 1643, | 1245, | 2953, | 754, | 1498 |
| 5 | 1644, | 1245, | 755, | 1114, | 1498 |
| 6 | 1644, | 1245, | 755, | 1114, | 1421 |
| 7 | 1644, | 2955, | 2929, | 2868, | 1421 |
| 8 | 1643, | 2963, | 1421, | 1271, | 1460 |
| 9 | 1642, | 1474, | 2934, | 1418, | 1452 |
| 10 | 1645, | 2962, | 1402, | 2931, | 1459 |
| 11 | 1643, | 1243, | 755, | 1497, | 1598 |
| 12 | 1643, | 1243, | 755, | 1498, | 1599 |
| 13 | 1643, | 1243, | 1498, | 754, | 1587 |
| 14 | 1644, | 1243, | 2927, | 754, | 1599 |
| 15 | 1643, | 1243, | 755, | 1498, | 1599 |
| 16 | | | | | |
| 17 | 1645, | 1242, | 1497, | 754, | 1419 |
| 18 | 1643, | 1246, | 755, | 1422, | 1498 |
| 19 | 1643, | 1245, | 754, | 1421, | 1498 |
| 20 | 1643, | 1245, | 754, | 1498, | 2931 |
| 21 | 1644, | 1245, | 2928, | 1489, | 754 |
| 22 | 1644, | 1245, | 755, | 1421, | 1498 |
| 23 | 1646, | 1244, | 1393, | 1195, | 756 |
| 24 | 1645, | 1245, | 1497, | 754, | 1600 |
| 25 | 1642, | 1246, | 1422, | 755, | 1498 |
| 26 | 1643, | 1245, | 1422, | 755, | 1498 |
| 27 | 1643, | 1245, | 2931, | 1498, | 1422 |
| 28 | 1643, | 1245, | 2928, | 754, | 1422 |
| 29 | 1643, | 1246, | 1498, | 1421, | 755 |
| 30 | 1645, | 1393, | 1246, | 1190, | 756 |
| 31 | | | | | |
| 32 | 1642, | 1245, | 1497, | 1600, | 754 |
| 33 | 1642, | 1245, | 1497, | 1600, | 754 |
| 34 | 1643, | 1245, | 2933, | 754, | 1421 |
| 35 | 1643, | 2928, | 1245, | 754, | 1497 |
| 36 | 1642, | 1245, | 755, | 1498, | ·1421 |
| 37 | 1641, | 1648, | 1393, | 1253, | 758 |
| 38 | 1643, | 1245, | 1497, | 1600, | 755 |
| 39 | 1642, | 1245, | 1497, | 2936, | 755 |
| 40 | 1043, | 1245, | 2935, | 1755, | 1498 |
| 41 | 1643, | 1245, | 2933, | 1498, | 754 |
| 42 | 1643, | 2929, | 1245, | 2856, | 754 |
| 43 | 1642, | 1245, | 754, | 2936, | 1498 |
| 44 | 1645, | 1248, | 1653, | 1394, | 1500 |
| 45 | 1643, | 1245, | 1497, | 755, | 1600 |
| 46 | 2924, | 1639, | 2852, | 1418, | 1449 |
| 47 | 1643, | 1474, | 1421, | 2961, | 2932 |
| 48 | 1644, | 2962, | 1420, | 1454, | 2931 |
| 49 | 1642, | 2959, | 2931, | 1421, | 2871 |
| 50 | 1643, | 2960, | 2932, | 1422, | 2871 |
| 51 | 1462, | 1642, | 2956, | 1419, | 761 |
| 52 | | | | | |
| 53 | 1646, | 1245, | 754, | 1497, | 1420 |
| 54 | 1644, | 1245, | 1492, | 755, | 1407 |
| 55 | 1644, | 1245, | 2947, | 754, | 1498 |
| 56 | 1644, | 1245, | 2948, | 1392, | 1497 |
| 57 | 1643, | 1245, | 2936, | 754, | 1498 |
| 58 | | | | | |
| 59 | | | | | |
| 60 | | | | | |
| 61 | | | | | |
| 62 | 1645, | 1249, | 1486, | 2951, | 1278 |
| 63 | | | | | |
| 64 | | | | | |

Die neuen Verbindungen zeichnen sich, allgemein ausgedrückt, durch eine hervorragende Wirksamkeit gegen ein breites Spektrum vonpflanzenpathogenen Pilzen, insbesondere aus der Klasse der Ascomyceten und Basidiomyceten, aus. Sie sind zum Teil systemisch wirksam und können, als Blatt- und Bodenfungizide eingesetzt werden.

Besonders interessant sind die fungiziden Verbindungen für die Bekämpfung einer Vielzahl von verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoraceatum und Sphaeroteca fuliginea an Kürbisgewächsen,
Podosphaeara leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten,
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach der Verwendungszwecken; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Tragerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin, Dimethylformamid) und Wasser; Trägerstoffe wie natürliche Gesteinsmehle, z. B. Kaoline, Tonerden, Talkum, Kreide und synthetische Gesteinsmehle (z. B. hochdisperse Kieselsäure, Silikate); Emulgiermittel, wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeiner zwischen 0,1 und 95, vorzugsweise zwischen 0,5 und 90 Gew.-% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 3 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 4 werden in einer Mischung gelöst, die aus 80 Gew.-Teilen Xylol, 10 Gew.-Teile des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 9 werden in einer Mischung gelöst, die aus 40 Gew.-Teilen Cyclohexanon,

30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 16 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Losung in Wasser erhält man eine wäßrige Dispersion.

V. 80 Gew.-Teile der Verbindung Nr. 21 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverformigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 22 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 28 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 29 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 31 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z. B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergroßerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyl-dithiocarbamat,
Zinkdimethyl-dithiocarbamat,
Zinkethylen-bis-dithiocarbamat,
Manganethylen-bis-dithiocarbamat,
Mangan-Zink-ethylendiamin-bis-dithiocarbamat,
Tetramethyl-thiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N' -Propylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat,
5-Nitro-isophthalsäure-di-isopropylester,

heterocyclische Strukturen, wie
2-Heptadecyl-2-imidazolin-acetat,
2,4-Dichlor-6-(o-chloranilino)-s-triazin,
0,0-Diethyl-phthalimido-phosphonothioat,
5-Amino-1-(bis-(dimethylamino)-phosphinyl)-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarboxylamino-benzimidazol
2-(Furyl-(2))-benzimidazol
2-(Thiazolyl-(4))-benzimidazol
N-(1,1,2,2-Tetrachlorethylthio)-tetrahydrophthalimid
N-Trichlormethylthio-tetrahydrophthalimid
N-Trichlormethylthio-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-Methyl-5,6-dihydro-4-H-pyran-3-carbonsäure-anilid
2-Methyl-furan-3-carbonsäure-anilid
2,5-Dimethyl-furan-3-carbonsäureanilid
2,4,5-Trimethyl-furan-3-carbonsäureanilid
2,5-Dimethyl-furan-3-carbonsäure-cyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecy-morpholin bzw. dessen Salze
N-[3-(p-tert.Butylphenyl)-2-methylpropyl)]-cis-2,6-dimethylmorpholin
N-[3-(p-tert.Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin
Bis-(p-Chlorphenyl)-pyridinmethanol,
1,2-Bis-(3-ethoxycarbonyl-2-thioureido)-benzol,
1,2-Bis-(3-methoxycarbonyl)-2-thioureido)-benzol

sowie verschiedene Fungizide, wie
Dodecylguanidinacetat,
3-(3(3,5-Dimethyl-2-oxycyclohexyl)-2-hydroxyethyl)-glutarimid,
Hexachlorbenzol,
DL-Methyl-N-(2,6-dimethyl-phenyl)-N-furoyl-(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl-(5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureamid
2-Cyano-N-(ethylaminocarbonyl)-2-methoximino)-acetamid
1-(2-(2,4-Dichlorphenyl)-pentyl)-1H-1,2,4-triazol
2,4-Difluor-α-(1H-1,2,4-triazolyl-1-methyl)-benzhydrylalkohol,
N-(n-Propyl)-N-(2,4,6-trichlorphenoxyethyl)-N'-imidazolylharnstoff.

Für die folgenden Versuche wurde als Vergleich der bekannte Wirkstoff Isoxazol-5-carbonsäure-N-(1-methoxycarbonyl-ethyl)-(2,6-dimethylanilid) (A) verwendet.

**Anwendungsbeispiel 1**

Wirksamkeit gegen Weizenmehltau

Blätter von in Töpfen gewachsenen Weizenkeimlingen der Sorte "Frühgold" werden mit wäßriger Spritzbrühe, die 80 % Wirkstoff und 20 % Emulgiermittel in der Trockensubstanz enthält, besprüht und nach 24 Stunden nach dem Antrocknendes Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit augestellt. Nach 7 Tagen wird das Ausmaß der Mehltauentwicklung ermittelt.

Das Ergebnis zeigt, daß die Verbindungen 3, 4, 9, 16, 21, 22, 28, 29, 31, 33, 40, 41, 42 bei der Anwendung als 0,025 %-ige Spritzbrühe eine bessere fungizide Wirkung (etwa 90 %) zeigen als der bekannte Wirkstoff A (50 %).

**Anwendungsbeispiel 2**

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Topfe für 24 Stunden bei 20 bis 22°C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70 % relativer Luftfeuchte aufgestellt. Nach 8 Tagen wird das Ausmaß der Rostpilzentwicklung auf den Blätter ermittelt.

Das Ergebnis zeigt, daß die Verbindungen 14, 27, 29, 30, 32, 33, 37, 39, 40, 41, 42, 43 bei der Anwendung als 0,025 %-ige Spritzbrühe eine bessere fungizide Wirkung (etwa 90 %) zeigen als der bekannte Wirkstoff A (30 %).

**Anwendungsbeispiel 3**

Wirksamkeit gegen Pyrenophora teres

Blätter von in Töpfen gewachsenen Gerstenkeimlingen der Sorte "Asse" werden im Zweiblattstadium mit wäßriger Spritzbrühe, die 80 % Wirkstoff und 20 % Emulgiermittel in der Trockensubstanz enthält, bis zur Tropfnässe besprüht. Am folgenden Tage werden die angetrockneten Pflanzen mit einer wäßrigen Sporensuspension von Pyrenophora teres inoculiert und für 7 Tage bei 17 bis 19°C und 95 % relativer Luftfeuchtigkeit weiter kultiviert. Dann wird das Ausmaß des Pilzbefalls ermittelt.

Das Ergebnis zeigt, daß die Verbindungen 7, 9, 26, 28, 29 bei der Anwendung als 0,05 %-ige Spritzbrühe eine gute fungizide Wirkung (etwa 90 %) zeigen.

**Anwendungsbeispiel 4**

Wirksamkeit gegen Septoria nodorum

Blätter von in Töpfen gewachsenen Weizenkeimlingen der Sorte "Jubilar" werden mit wäßriger Spritzbrühe, die 80 % Wirkstoff und 20 % Emulgiermittel in der Trockensubstanz enthält, besprüht, nach dem Antrocknen des Spritzbelages abgeschnitten und in Schalen mit wäßriger Benzimidazollösung (25 ppm) ausgelegt. Dann werden die Blätter mit einer wäßrigen Sporensuspension von Septoria nodorum infiziert und abgedeckt. Nach 7-tägigem Stehen bei 20 bis 22°C wird das Ausmaß der Pilzentwicklung ermittelt.

Das Ergebnis zeigt, daß die Verbindungen 3, 14, 26, 27, 29, 32, 33, 40 bei der Anwendung als 0,1 %-ige Spritzbrühe eine gute fungizide Wirkung (etwa 90 %) zeigen.

**Patentansprüche**

1. Verbindungen der allgemeinen Formel I

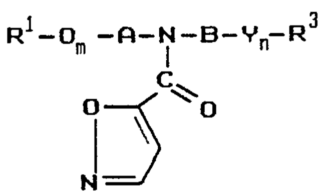

(I)

in der

$R^1$ einen gegebenenfalls ein- bis dreifach durch Halogen, $C_1$-$C_5$-Alkyl, $C_1$-$C_2$-Alkoxy, $C_1$-$C_2$-Alkylthio, $C_1$-$C_2$-Halogenalkyl, Nitro, Phenyl, Halogenphenyl, Phenoxy, Halogenphenoxy substituierten Phenylrest oder einen gegebenenfalls ein- bis dreifach durch $C_1$-$C_3$-Alkyl substituierten $C_1$-$C_8$-Alkylrest, einen gegebenenfalls ein- bis dreifach

durch $C_1$-$C_3$-Alkyl substituierten $C_3$-$C_8$-Cycloalkylrest oder einen gegebenenfalls ein- bis dreifach durch Halogen oder $C_1$-$C_4$-Alkyl substituierten Benzylrest bedeutet;

Y ein Sauerstoffatom oder die Gruppe $NR^2$ bedeutet,
$R^2$ einen Alkylrest mit 1 bis 4 C-Atomen bedeutet;
m und n unabhängig voneinander für die Zahlen 0 und 1 stehen;
A für einen Alkylenrest mit 2 bis 10 Kohlenstoffatomen steht;
B für einen Alkylenrest mit einem bis 10 Kohlenstoffatomen oder einen Cycloalkylenrest mit 5 bis 8 Kohlenstoffatomen steht und
$R^3$ einen Phenylrest, einen Halogenphenylrest, einen Alkylrest mit 1 bis 10 Kohlenstoffatomen oder ein Wasserstoffatom bedeutet.

2. Fungizid, *gekennzeichnet durch* einen Gehalt an einem üblichen Trägerstoff und einer Verbindung der allgemeinen Formel I

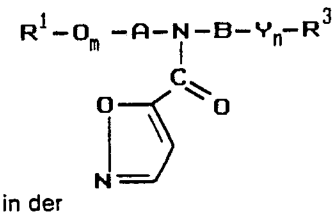

(I)

in der

$R^1$ einen gegebenenfalls ein- bis dreifach durch Halogen, $C_1$-$C_5$-Alkyl, $C_3$-$C_2$-Alkoxy, $C_1$-$C_2$-Alkylthio, $C_1$-$C_2$-Halogenalkyl, Nitro, Phenyl, Halogenphenyl, Phenoxy, Halogenphenoxy substituierten Phenylrest oder einen gegebenenfalls ein- bis dreifach durch $C_1$-$C_3$-Alkyl substituierten $C_1$-$C_8$-Alkylrest, einen gegebenenfalls ein- bis dreifach durch $C_1$-$C_3$-Alkyl substituierten $C_3$-$C_8$-Cycloalkylrest oder einen gegebenenfalls ein- bis dreifach durch Halogen oder $C_1$-$C_4$-Alkyl substituierten Benzylrest bedeutet;
Y ein Sauerstoffatom oder die Gruppe $NR^2$ bedeutet,
$R^2$ einen Alkylrest mit 1 bis 4 C-Atomen bedeutet;
m und n unabhängig voneinander für die Zahlen 0 und 1 stehen;
A für einen Alkylenrest mit 2 bis 10 Kohlenstoffatomen steht;
B für einen Alkylenrest mit einem bis 10 Kohlenstoffatomen oder einen Cycloalkylenrest mit 5 bis 8 Kohlenstoffatomen steht und
$R^3$ einen Phenylrest, einen Halogenphenylrest, einen Alkylrest mit 1 bis 10 Kohlenstoffatomen oder ein Wasserstoffatom bedeutet.

3. Verfahren zur Bekämpfung von Pilzen, *dadurch gekennzeichnet*, daß man die Pilze, ihren Lebensraum, Saatgüter, Pflanzen, Boden oder Materialien mit einer fungizid wirksamen Menge einer Verbindung der allgemeinen Formel I

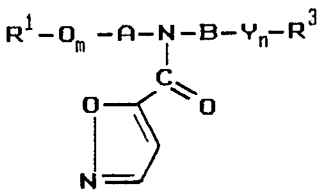

(I)

in der

$R^1$ einen gegebenenfalls ein- bis dreifach durch Halogen, $C_1$-$C_5$-Alkyl, $C_1$-$C_2$-Alkoxy, $C_1$-$C_2$-Alkylthio, $C_1$-$C_2$-Halogenalkyl, Nitro, Phenyl, Halogenphenyl, Phenoxy, Halogenphenoxy substituierten Phenylrest oder einen gegebenenfalls ein- bis dreifach durch $C_1$-$C_3$-Alkyl substituierten $C_1$-$C_8$-Alkylrest, einen gegebenenfalls ein- bis dreifach durch $C_1$-$C_3$-Alkyl substituierten $C_3$-$C_8$-Cycloalkylrest oder einen gegebenenfalls ein- bis dreifach durch Halogen oder $C_1$-$C_4$-Alkyl substituierten Benzylrest bedeutet;
Y ein Sauerstoffatom oder die Gruppe $NR^2$ bedeutet, $R^2$einen Alkylrest mit 1 bis 4 C-Atomen bedeutet;
m und
n unabhängig voneinander für die Zahlen 0 und 1 stehen;
A für einen Alkylenrest mit 2 bis 10 Kohlenstoffatomen steht;
B für einen Alkylenrest mit einem bis 10 Kohlenstoffatomen oder einen Cycloalkylenrest mit 5 bis 8 Kohlenstoffatomen steht und
$R^3$ einen Phenylrest, einen Halogenphenylrest, einen Alkylrest mit 1 bis 10 Kohlenstoffatomen oder ein Wasserstoffatom bedeutet, behandelt.

4. Verwendung von 5-Isoxazolcarbonsäureamiden der Formel I gemäß Anspruch 1 zur Bekämpfung von Pilzen.

5. Verfahren zur Herstellung von Fungiziden, *dadurch gekennzeichnet*, daß man 5-Isoxazolcarbonsäureamide der Formel I gemäß Anspruch 1 mit festen oder flüssigen Trägerstoffen und/oder oberflächenaktiven Mitteln mischt.

**Claims**

1. A compound of the formula I

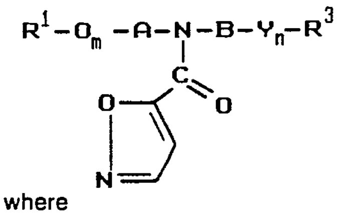

(I)

where

$R^1$ is phenyl which is unsubstituted or monosubstituted to trisubstituted by halogen, $C_1$-$C_5$-alkyl, $C_1$- or $C_2$-alkoxy, $C_1$- or $C_2$-alkylthio, $C_1$- or $C_2$-haloalkyl, nitro, phenyl, halophenyl, phenoxy or halophenoxy, or is $C_1$-$C_8$-alkyl which is unsubstituted or monosubstituted to trisubstituted by $C_1$-$C_3$-alkyl, or is $C_3$-$C_8$-cycloalkyl which is unsubstituted or monosubstituted to trisubstituted by $C_1$-$C_3$-alkyl, or is benzyl which is unsubstituted or monosubstituted to trisubstituted by halogen or $C_1$-$C_4$-alkyl,

Y is oxygen or a group $NR^2$,
$R^2$ is alkyl of 1 to 4 carbon atoms,
m and n independently of one another are each 0 or 1,
A is alkylene of 2 to 10 carbon atoms,
B is alkylene of one to 10 carbon atoms or cycloalkylene of 5 to 8 carbon atoms and
$R^3$ is phenyl, halophenyl, alkyl of 1 to 10 carbon atoms or hydrogen.

2. A fungicide which contains a conventional carrier and a compound of the formula I

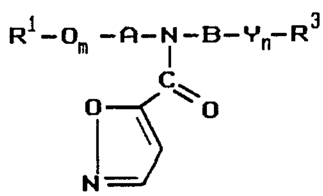

(I)

where

$R^1$ is phenyl which is unsubstituted or monosubstituted to trisubstituted by halogen, $C_1$-$C_5$-alkyl, $C_1$- or $C_2$-alkoxy, $C_1$- or $C_2$-alkylthio, $C_1$- or $C_2$-haloalkyl, nitro, phenyl, halophenyl, phenoxy or halophenoxy, or is $C_1$-$C_8$-alkyl which is unsubstituted or monosubstituted to trisubstituted by $C_1$-$C_9$-alkyl, or is $C_3$-$C_8$-cycloalkyl which is unsubstituted or monosubtituted to trisubstituted by $C_1$-$C_3$-alkyl, or is benzyl which is unsubstituted or monosubstituted to trisubstituted by halogen or $C_1$-$C_4$-alkyl,
Y is oxygen or a group $NR^2$,
$R^2$ is alkyl of 1 to 4 carbon atoms,
m and n independently of one another are each 0 or 1,
A is alkylene of 2 to 10 carbon atoms,
B is alkylene of one to 10 carbon atoms or cycloalkylene of 5 to 8 carbon atoms and
$R^3$ is phenyl, halophenyl, alkyl of 1 to 10 carbon atoms or hydrogen.

3. A method for controlling fungi, wherein the fungi, their habitat, seed, plants, soil or materials are or is treated with a fungicidally effective amount of a compound of the formula I

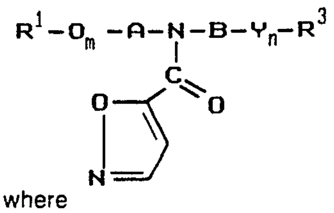

(I)

where

$R^1$ is phenyl which is unsubstituted or monosubstituted to trisubstituted by halogen, $C_1$-$C_5$-alkyl, $C_1$- or $C_2$-alkoxy, $C_1$- or $C_2$-alkylthio, $C_1$- or $C_2$-haloalkyl, nitro, phenyl, halophenyl, phenoxy or halophenoxy, or is $C_1$-$C_8$-alkyl which is unsubstituted or monosubstituted to trisubstituted by $C_1$-$C_3$-alkyl, or is $C_3$-$C_8$-cycloalkyl which is unsubstituted or monosubstituted to trisubstituted by $C_1$-$C_3$-alkyl, or is benzyl which is unsubstituted or monosubstituted to trisubstituted by halogen or $C_1$-$C_4$-alkyl,
Y is oxygen or a group $NR^2$,
$R^2$ is alkyl of 1 to 4 carbon atoms,
m and n independently of one another are each 0 or 1,
A is alkylene of 2 to 10 carbon atoms,
B is alkylene of one to 10 carbon atoms or cycloalkylene of 5 to 8 carbon atoms and
$R^3$ is phenyl, halophenyl, alkyl of 1 to 10 carbon atoms or hydrogen.

4. Use of a 5-isoxazolecarboxamide of the formula I as claimed in claim 1 for controlling fungi.

5. A process for the preparation of a fungicide wherein a 5-isoxazolecarboxamide of the formula I as claimed in claim 1 is mixed with solid or liquid carriers and/or surfactants.

**Revendications**

1. Composés de la formule générale I

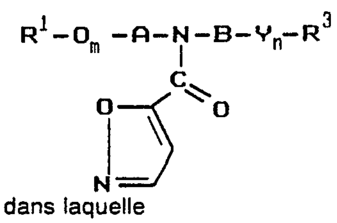

(I)

dans laquelle

$R^1$ représente un reste phényle, éventuellement substitué une ou trois fois par halogène, alkyle en $C_1$-$C_5$, alcoxy en $C_1$-$C_2$, alkylthio en $C_1$-$C_2$, halogénoalkyle en $C_1$-$C_2$, nitro, phényle, halogénophényle, phénoxy, halogénophénoxy ou un reste alkyle en $C_1$-$C_8$ éventuellement substitué une ou trois fois par alkyle en $C_1$-$C_8$, un reste cycloalkyle en $C_3$-$C_8$ éventuellement substitué une ou trois fois par alkyle en $C_1$-$C_3$ ou un reste benzyle éventuellement substitué une ou trois fois par halogène ou alkyle en $C_1$-$C_4$,
Y représente un atome d'oxygène ou le groupe $NR^2$,
$R^2$ représente un reste alkyle ayant 1 à 4 atomes C;
m et n sont mis, indépendamment l'un de l'autre, pour les nombres 0 et 1;
A est mis pour un reste alkylène ayant 2 à 10 atomes de carbone;
B est mis pour un reste alkylène ayant 1 à 10 atomes de carbone ou un reste cycloalkylène ayant 5 à 8 atomes de carbone et
$R^3$ représente un reste phényle, un reste halogénophényle, un reste alkyle ayant 1 à 10 atomes de carbone ou un atome d'hydrogène.

2. Fongicide, caractérisé par le fait qu'il contient, sur une matière de support usuelle, un composé de la formule générale I

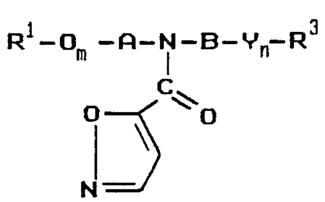

(I)

dans laquelle

$R^1$ représente un reste phényle, éventuellement substitué une ou trois fois par halogène, alkyle en $C_1$-$C_5$, alcoxy en $C_1$-$C_2$, alkylthio en $C_1$-$C_2$, halogénoalkyle en $C_1$-$C_2$, nitro, phényle, halogénophényle, phénoxy, halogénophénoxy ou un reste alkyle en $C_1$-$C_8$ éventuellement substitué une ou trois fois par alkyle en $C_1$-$C_3$, un reste cycloalkyle en $C_3$-$C_8$ éventuellement substitué une ou trois fois par alkyle en $C_1$-$C_3$ ou un reste benzyle éventuellement substitué une ou trois fois par halogène ou alkyle en $C_1$-$C_4$,
Y représente un atome d'oxygène ou le groupe $NR^2$,
$R^2$ représente un reste alkyle ayant 1 à 4 atomes C;
m et n sont mis, indépendamment l'un de l'autre, pour les nombres 0 et 1;
A est mis pour un reste alkylène ayant 2 à 10 atomes de carbone;
B est mis pour un reste alkylène ayant 1 à 10 atomes de carbone ou un reste cycloalkylène ayant 5 à 8 atomes de carbone et
$R^3$ représente un reste phényle, un reste halogénophényle, un reste alkyle ayant 1 à 10 atomes de carbone ou un atome d'hydrogène.

3. Procédé pour lutter contre des champignons caractérisé par le fait que l'on traite les champignons, leur biotope, semences, plantes, sols ou matériaux avec une quantité efficace du point de vue fongicide d'un composé de la formule générale I

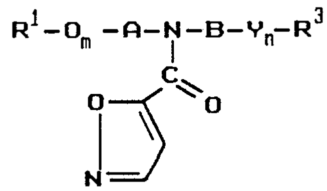

(I)

dans laquelle

$R^1$ représente un reste phényle, éventuellement substitué une ou trois fois par halogène, alkyle en $C_1$-$C_5$, alcoxy en $C_1$-$C_2$, alkylthio en $C_1$-$C_2$, halogénoalkyle en $C_1$-$C_2$, nitro, phényle, halogénophényle, phénoxy, halogénophénoxy ou un reste alkyle en $C_1$-$C_8$ éventuellement substitué une ou trois fois par alkyle en $C_1$-$C_3$, un reste cycloalkyle en $C_3$-$C_8$, éventuellement substitué une ou trois fois par alkyle en $C_1$-$C_3$ ou un reste benzyle éventuellement substitué une ou trois fois par halogène ou alkyle en $C_1$-$C_4$,
Y représente un atome d'oxygène ou le groupe $NR^2$,
$R^2$ représente un reste alkyle ayant 1 à 4 atomes C;
m et n sont mis, indépendamment l'un de l'autre, pour les nombres 0 et 1;
A est mis pour un reste alkylène ayant 2 à 10 atomes de carbone;
B est mis pour un reste alkylène ayant 1 à 10 atomes de carbone ou un reste cycloalkylène ayant 5 à 8 atomes de carbone et
$R^3$ représente un reste phényle, un reste halogénophényle, un reste alkyle ayant 1 à 10 atomes de carbone ou un atome d'hydrogène.

4. Utilisation d'amides d'acide 5-isoxazolcarboxylique de formule I selon la revendication 1 pour la lutte contre les champignons.

5. Procédé de préparation de fongicides, caractérisé par le fait que l'on mélange de l'amide d'acide 5-isoxazolcarboxylique de formule I selon la revendication 1 avec des matières de support solides ou liquides et/ou des agents tensio-actifs.